# FASCICULE DE BREVET EUROPEEN

(11) **EP 1 151 754 B1**
(45) Date de publication et mention de la délivrance du brevet: **01.02.2006**
(21) Numéro de dépôt: 01401165.4
(22) Date de dépôt: 04.05.2001
(51) Int. Cl.: A61K 35/20, A61P 29/00, A23C 9/00

(54) **Utilisation d'une fraction du lait d'équidé choisie parmi du lait écrémé, du lactosérum et du caséinate de sodium ayant une activité anti-inflammatoire**
Verwendung einer Stutenmilchfraktion ausgewählt aus Magermilch, Molke und Natriumcaseinat mit entzündungshemmender Wirkung
Use of a equine milk fraction selected from skim milk, lactoserum and sodium caseinate having anti-inflammatory activity

(30) Priorité: 05.05.2000 FR 0005803
(43) Date de publication de la demande: 07.11.2001
(73) Titulaire: Ballestra, François, 83700 Saint-Raphael (FR)
(72) Inventeur: Ballestra, François, 83700 Saint-Raphael (FR)
(74) Mandataire: Michelet, Alain

(56) Documents cités:
- WO-A-94/16675
- WO-A-95/01100
- WO-A-98/00149
- DE-A- 1 961 990
- FR-A- 2 783 172
- GB-A- 1 496 671
- PATENT ABSTRACTS OF JAPAN vol. 012, no. 450 (C-547), 25 novembre 1988 (1988-11-25) & JP 63 173559 A (NICHIEI CHEM:KK), 18 juillet 1988 (1988-07-18)
- GRAZIOSO CARLOS F ET AL: "Antiinflammatory effects of human milk on chemically induced colitis in rats." PEDIATRIC RESEARCH, vol. 42, no. 5, 1997, pages 639-643, XP000971828 ISSN: 0031-3998
- GAROFALO ROBERTO P ET AL: "Expression of functional immunomodulatory and anti-inflammatory factors in human milk." CLINICS IN PERINATOLOGY, vol. 26, no. 2, juin 1999 (1999-06), pages 361-377, XP000971816 ISSN: 0095-5108
- DATABASE WPI Section Ch, Week 199503 Derwent Publications Ltd., London, GB; Class B04, AN 1995-019278 XP002157620 & JP 06 306099 A (MORINAGA MILK IND CO LTD), 1 novembre 1994 (1994-11-01)

## Description

La présente invention concerne l'utilisation d'une fraction du lait d'équidé choisie parmi du lait écrémé, du lactosérum, et du caséinate de sodium pour la fabrication d'une composition possédant une activité anti-inflammatoire.

L'invention est également relative à des compositions comprenant une fraction du lait d'équidé choisie parmi du lait écrémé, du lactosérum, et du caséinate de sodium telle qu'une composition pharmaceutique, une composition cosmétique ou encore une composition destinée à l'alimentation de l'homme ou de l'animal.

Les laits d'équidés, tels que la jument ou l'ânesse ont une composition en matières organiques et minérales originale. Ils sont généralement considérés comme les laits ayant la composition en substances organique et minérale la plus proche de celle du lait de femme.

Ainsi, la teneur en lactose est d'environ 60 g/kg pour le lait d'ânesse et d'environ 73 g/kg pour le lait de jument, pour une teneur en protéines respectivement d'environ 16 g/kg et 20,3 g/kg. Le lait d'équidé possède en outre une teneur moyenne en matière minérale d'environ 5 g/kg, une teneur en caséine d'environ 8 à 10 g/kg et une teneur en protéines du lactosérum de 8 à 9 g/kg. Les données d'analyse de la composition des laits d'ânesse et de jument peuvent par exemple être retrouvées dans les publications de El Salam et al. (1992, Proc. 5th Egyptian Conf. Dairy Sci. & Techn., PP. 281-287), KUDRYASHOV et al. (1965, Dairy. Sci. Abstr., Volume 28: 2299) et OCHIRKHUYAG (1999, « Caractérisation des caséines du lait de jument. Propriétés biologiques du lait équin fermenté (Koumiss) », Université de Nantes, pp.1-95 (thèse de doctorat en Sciences).

Dans sa globalité, la composition du lait d'ânesse est donc comparable à celle du lait de jument. En particulier, du fait de leur faible teneur en matière grasse et en matière azotée, les laits d'équidés ont une teneur en matière sèche relativement faible comparée aux laits de vache ou de femme (en moyenne 90 g/kg contre 120 g/kg).

Par conséquent, leur valeur calorique est considérablement abaissée par rapport aux laits des autres mammifères, le lactose étant le principal composant énergétique dans le lait d'équidé.

Les laits d'équidés, tels que le lait de jument et d'ânesse, ont été moins étudiés que les laits de vache, de chèvre, de brebis et de femme.

Les premières études sur la composition du lait d'ânesse datent de la fin du 19ème siècle. Ces études ont montré que le lait d'ânesse est un lait proche de celui de la femme. De plus, le lait d'ânesse serait facilement assimilable du fait qu'il contient une teneur en vitamine C élevée (6-8,5 mg/100 ml) et que les protéines du lait d'ânesse seraient plus facilement digérées par la trypsine que celles du lait de vache.

L'utilisation du lait d'équidés à des fins médicales a été suggérée dès l'antiquité. Il a notamment été proposé dans l'état de la technique d'utiliser le lait d'ânesse pour les soins de la peau, par exemple comme décrit dans la demande de brevet français n°93 08375, qui divulgue un procédé de conservation du lait d'ânesse. De même, la demande de brevet français publiée sous le n°2.783.172 suggère l'utilisation du lait d'équidé, notamment du lait de jument ou du lait d'ânesse, pour traiter les affections bucco-dentaires.

Il a également été proposé d'utiliser du colostrum d'équidé pour la préparation de compositions dermo-cosmétiques, comme par exemple dans la demande PCT publiée sous le n°WO 94/16.675. Le colostrum est un liquide physiologique sécrété par les glandes mammaires des mammifères durant la première semaine suivant la mise bas et qui possède une composition très différente de celle du lait: Le colostrum se caractérise par rapport au lait par une teneur élevée en protéines (50 à 150 g/kg) et en sels minéraux (10 à 12 g/kg). L'accroissement de la teneur en protéines est due à la présence d'un taux élevé d'immunoglobulines G nécessaires à la protection immunitaire du jeune. Le colostrum se caractérise aussi par une teneur en lactose abaissée 60% de la teneur du lait mature (A Paris-Science du lait, 1984).

Il a désormais été montré selon l'invention que, de manière surprenante, des fractions du lait d'équidé choisie parmi du lait écrémé, du lactosérum, et du caséinate de sodium sont capables d'inhiber la production par les cellules d'une cytokine pro-inflammatoire, et plus spécifiquement la production d'interleukine-1.

L'interleukine-1 est une cytokine dite « inflammatoire ». L'activité pro-inflammatoire de l'interleukine-1 peut être observée avec des concentrations faibles de cette cytokine, de l'ordre du picomolaire et même du fentomolaire, étant observé que des récepteurs spécifiques de l'interleukine-1 sont exprimés par la plupart des cellules.

Il est distingué deux types d'interleukine-1, respectivement l'interleukine-1α et l'interleukine-1β. L'interleukine-1α est produite de manière constitutive par diverses cellules épithéliales, les kératinocytes de la peau et du cerveau, ainsi que par des cellules du système immunitaire dont les monocytes/macrophages et certains polynucléaires tels que les polynucléaires neutrophiles.

L'interleukine-1 contribue aux fonctions de croissance et de réparation des cellules, en particulier des cellules épithéliales et des kératinocytes. Durant le développement d'une réponse immunitaire, par exemple à la suite d'une infection, l'interleukine-1 est produite et peut provoquer une situation inflammatoire.

Par exemple, une réponse inflammatoire excessive des kératinocytes a été expérimentalement provoquée par une surexpression des récepteurs à l'interleukine-1 chez des souris transgéniques, une telle réponse inflammatoire étant caractérisée par une hyperplasie épidermique et des infiltrations cellulaires inflammatoires dermiques aiguës (GROVES et al., 1996, J. Clin. Invest., vol.98 (2):336-344). De même, on a montré que l'interleukine-1 est capable de stimuler la prolifération des kératinocytes de la peau comparable à celle observée dans diverses maladies de la peau telles que l'épidermite hyperproliférative retrouvée dans certains types de maladies inflammatoires de la peau (RISTOW, 1987, Proc. Natl. Acad. Sci. USA, vo1.84 (7): 1940-1944).

Il a également été observé que l'interleukine-1α était produite à un niveau faible par les cellules localisées au niveau d'un tissu en cicatrisation anormalement lente, par exemple chez des patients soumis à un stress psychologique (GLASER R. et al., 1999, Arch. Gen Psychiatry, vol.56 (5): 450-456).

La production en excès d'interleukine-1 et sa libération en excès par les kératinocytes semble être impliquée dans le développement de maladies de peau telles que le psoriasis, les maladies bulleuses et d'autres maladies de peau (MIZUTANI H., 1998, J. dermatol Sci. Vol. 20-(1): 63-71). Un rôle important de l'interleukine-1 dans le développement de périodontite expérimentale a également été montré (GRAVES DT, 1999, Clin. Infect. Dis, vol.28(3): 482-490).

Par ailleurs, on a observé que des agressions de la peau, par exemple par des polluants tels que des particules issues de la combustion de carburant diesel, ou encore le formaldéhyde ou l'oxyde nitrique étaient capables d'augmenter la production d'II-1 par des kératinocytes dermiques humains, provoquant ainsi des inflammations cutanées (USHIO H. et al., 1999, Toxicol. Lett., vol. 105(1): 17-24; ANDREW PJ, 1999, Biochem. Pharmacol., vol. 57 (12):1423-1429).

D'autres types d'agressions de la peau ou des cellules de la cornée de l'oeil, telle qu'une exposition à un rayonnement ultraviolet, induisent également une augmentation de la production d'interleukine-1 par ces tissus, provoquant une inflammation (KUPPER TS et.al., 1987, J. Clin. Invest., vol.80(2): 430-436; KENNEDY M. et al., Invest. Ophtalmol vis Sci. Vol.38(12):2483-2491).

Il ressort de ce qui précède qu'un effet d'inhibition de la production de l'interleukine-1 et plus particulièrement de l'interleukine-1α, peut être assimilé à un effet anti-inflammatoire, qui peut être systémique ou au contraire local, par exemple au niveau de la peau.

Le demandeur a désormais montré que des fractions de lait d'équidé choisie parmi du lait écrémé, du lactosérum, et du caséinate de sodium possédaient une activité inhibitrice de la production d'interleukine-1 tout particulièrement d'interleukine-1α par les cellules.

Il est ainsi fourni selon l'invention, des moyens possédant une activité anti-inflammatoire, qui ont été obtenus à partir du lait d'équidé.

Un premier objet de l'invention concerne l'utilisation d'une fraction du lait d'équidé choisie parmi du lait écrémé, du lactosérum et du caséinate de sodium pour la fabrication d'une composition possédant une activité anti-inflammatoire.

Par « lait d'équidé », on entend selon l'invention, un lait produit par un mammifère du genre *Equus,* auquel appartiennent la jument et l'ânesse.

Par « fraction de lait » au sens de l'invention, on entend tout produit dérivé préparé à partir du lait entier, par exemple par centrifugation, y compris ultracentrifugation, filtration, y compris microfiltration et ultrafiltration, ou encore séparation des différents constituants, par exemple sur des colonnes chromatographiques telles que les colonnes chromatographiques d'exclusion de taille, d'échange d'anions ou de cations ou encore des colonnes pour chromatographie en phase inverse.

Parmi les fractions du lait, on peut citer tout particulièrement le lait écrémé, le lactosérum ou encore le caséinate tel que le caséinate de sodium.

La fraction de lait écrémé peut être obtenue à partir du lait entier d'équidé selon toute technique connue de l'homme du métier, par exemple par centrifugation, microfiltration ou l'ultrafiltration.

Le lactosérum peut être obtenu par exemple par l'ultracentrifugation ou par microfiltration tangentielle sur membrane ayant un diamètre de pores de l'ordre de 0.1 µm.

Le caséinate de sodium peut être préparé par acidification du lait entier ou du lait écrémé, entraînant une précipitation et donc une concentration de caséine qui est ainsi facilement récupérée, le cas échéant, après dialyse contre de l'eau distillée. La caséine micellaire peut être obtenue par microfiltration et diafiltration.

Les différentes fractions du lait d'équidé ci-dessus peuvent se présenter sous la forme d'une solution liquide.

Avantageusement, la dernière étape de préparation de chacune des fractions du lait d'équidé consiste en un séchage ou une lyophilisation selon des techniques bien connues de l'homme du métier dans le but d'obtenir ladite fraction du lait d'équidé sous la forme d'une poudre.

On entend par « microfiltration » une filtration du lait d'équidé selon toute technique connue de l'homme du métier et mettant en oeuvre une membrane filtrante ayant un seuil de coupure compris entre 0,05 µm et 0,5 µm.

On entend par « ultrafiltration » une filtration du lait d'équidé par toute technique connue de l'homme du métier et mettant en oeuvre une membrane filtrante ayant un seuil de coupure compris entre 5 Kda et 150 kDa (kdaltons).

Comme déjà mentionné précédemment, l'inhibition de la production d'interleukine-1 peut avoir un effet bénéfique sur des situations pathologiques, notamment de la peau telles que les épidermites, le psoriasis, les oedèmes ou encore les inflammations systémiques provoquées par une infection bactérienne, fongique ou virale et donc un effet préventif ou curatif thérapeutique de telles pathologies inflammatoires.

En outre, l'inhibition de la production d'interleukine-1 provoquée par l'utilisation d'une fraction de lait d'équidé conformément à l'invention permet également une réduction des effets délétères, notamment inflammatoires, provoqués par l'exposition de la peau à des agents agressifs externes, comme par exemple des agents chimiques ou encore le rayonnement ultraviolet.

De plus, l'inhibition de la production d'interleukine-1 grâce à l'utilisation des fractions du lait d'équidé conformément à l'invention peut permettre de stabiliser des situations inflammatoires chroniques, par exemple lorsque la fraction du lait d'équidé est régulièrement absorbée oralement, par exemple simultanément à la prise alimentaire ou encore lorsque cette fraction du lait d'équidé est incorporée dans les aliments.

L'invention a donc également trait à l'utilisation d'une fraction du lait d'équidé choisie parmi du lait écrémé, du lactosérum, et du caséinate de sodium pour la préparation d'une composition pharmaceutique, d'une composition cosmétique ou d'une composition alimentaire fonctionnelle, encore appelée « alicament » dans le domaine de la « nutraceutique ».

L'invention a encore pour objet une composition caractérisée en ce qu'elle comprend une fraction d'un lait d'équidé choisie parmi du lait écrémé, du lactosérum, et du caséinate de sodium possédant une activité anti-inflammatoire.

La fraction du lait d'équidé est choisie parmi du lait écrémé, du lactosérum, et du caséinate de sodium.

Selon un premier aspect, la composition ci-dessus est une composition pharmaceutique.

Une telle composition pharmaceutique peut contenir la fraction du lait d'équidé à titre de principe actif unique ou au contraire contenir une association de principes actifs incluant le lait d'équidé. Il peut être avantageux de combiner, dans une même composition pharmaceutique, un principe actif dont on connaît les propriétés pro-inflammatoires indésirables avec une fraction du lait d'équidé selon l'invention. Une telle composition pharmaceutique contient de préférence de 1% à 80% en poids de la fraction riche du lait d'équidé, par rapport au poids total de la composition, préférentiellement de 5% à 50% en poids et de manière tout à fait préférée de 10% à 40% en poids, par rapport au poids de ladite composition.

Une composition pharmaceutique selon l'invention est avantageusement produite en formulant la fraction riche du lait, en général sous la forme d'une poudre, dans une forme de dosage comprenant au moins un excipient ou véhicule pharmaceutiquement acceptable. Pour préparer une composition pharmaceutique selon l'invention, les véhicules pharmaceutiquement acceptables peuvent être indifféremment solides ou liquides.

De préférence, une composition pharmaceutique selon l'invention est caractérisée en ce qu'il s'agit d'une formulation pour l'administration orale.

Des formes de dosage solide pour administration orale incluent les gélules, les comprimés, les pilules, les poudres et les granules.

D'une manière générale, les véhicules pharmaceutiquement acceptables utiles pour la préparation d'une composition pharmaceutique selon l'invention peuvent être retrouvés par l'homme du métier dans l'ouvrage suivant: « Remingston's Pharmaceutical Sciences, 17ème édition, Mack Publishing Company, Easton, Pen, 1985).

L'invention a encore pour objet une composition cosmétique, en particulier pour les soins de la peau, comprenant une fraction du lait d'équidé choisie parmi du lait écrémé, du lactosérum, et du caséinate de sodium et possédant une activité anti-inflammatoire.

Une composition cosmétique selon l'invention comprendra avantageusement la fraction du lait d'équidé en association avec un véhicule cosmétiquement acceptable, tel qu'un agent diluant ou un agent dispersant. Le véhicule cosmétiquement acceptable peut être aqueux, anhydre ou sous la forme d'une émulsion.

De préférence, les compositions sont sous la forme d'une solution aqueuse ou encore sous la forme d'une émulsion, de préférence une émulsion eau-dans-huile ou huile-dans-eau. L'eau peut être comprise dans des quantités allant de 5 à 99%, de préférence de 20 à 70% et de manière tout à fait préférée de 35 à 65% en poids, par rapport au poids total de la composition cosmétique.

Une telle composition cosmétique peut également comprendre des solvants volatils tels que des alcanols monohydriques en C₁ à C₃, tels que l'éthanol, le méthanol ou l'alcool isopropylique. La quantité d'alcanol monohydrique est comprise entre 1 et 70%, de préférence entre 10 et 50% et de manière tout à fait préférée entre 15 et 40% en poids, par rapport au poids total de la composition cosmétique.

Une telle composition cosmétique peut également contenir un matériau émollient qui peut être utilisé comme véhicule cosmétiquement acceptable, tel que des huiles de silicone et des esters synthétiques. La quantité d'agent émollient est avantageusement comprise entre 0,1 et 50%, de préférence entre 1 et 20% en poids, par rapport au poids total de la composition cosmétique.

A titre illustratif, une composition cosmétique comprenant une fraction d'un lait d'équidé conformément à l'invention peut être utilisée comme composition d'apaisement après une exposition aux rayons ultraviolets, par exemple après exposition au soleil ou à des rayons ultraviolets d'un dispositif de bronzage artificiel.

L'invention concerne encore une composition pour l'alimentation fonctionnelle de l'homme ou de l'animal comprenant une fraction d'un lait d'équidé choisie parmi du lait écrémé, du lactosérum, et du caséinate de sodium et possédant une activité anti-inflammatoire.

Un tel aliment fonctionnel comprendra avantageusement de 5% à 80%, de préférence de 10% à 60% et de manière tout à fait préférée de 12% à 30% en poids de la fraction riche d'un lait d'équidé, par rapport au poids total de la composition alimentaire fonctionnelle ou « alicament ». La fraction riche du lait peut être mélangée à tout type de produit alimentaire selon des techniques bien connues de l'homme du métier, tel qu'une solution de sucre (lactose ou saccharose) ou une solution saline isotonique.

L'invention est en outre illustrée, sans pour autant être limitée, par les exemples suivants.

### EXEMPLE 1:

### Préparation de la fraction de lait écrémé à partir d'un lait d'équidé.

Le matériau de départ est un mélange de lait de sept ânesses.

Le mélange est d'abord porté à la température de 30°C pendant 10 minutes puis l'étape d'écrémage est réalisée par centrifugation à 1000g pendant cinq minutes à 10°C, suivie d'un séjour à -20°C pendant 45 minutes afin de solidifier la crème ainsi séparée.

Le lait écrémé est ensuite lyophilisé à l'aide d'un lyophilisateur du type CS-10 de la Société Lyofroid, selon les paramètres suivants:
- température de congélation : - 25°C , durée 1 h
- vide moyen : 0.2 bars
- durée de la sublimation: 40 h pour une épaisseur de solution congelée de 1.5 cm.

La poudre est conservée dans un environnement sec.

### EXEMPLE 2 :

### Préparation de la fraction de lactosérum à partir de lait d'équidé.

Un mélange de lait d'ânesse est soumis à une ultracentrifugation à 100.000 g pendant une heure à 30°C. La fraction de lactosérum constitue le surnageant du produit issu de l'ultracentrifugation.

Le lactosérum est ensuite stérilisé par filtration à l'aide d'un dispositif de filtration équipé d'une membrane de taille moyenne de pore de 0,22µm du type « oméga » commercialisé par la Société FILTRON.

Le lactosérum stérile est ensuite lyophilisé dans un lyophilisateur CS-10 selon les paramètres identiques à ceux de l'exemple 1.

### EXEMPLE 3:

### Préparation du caséinate de sodium à partir de lait d'équidé.

Du lait écrémé est préparé à partir d'un mélange de laits d'ânesse, conformément à l'exemple 1.

Un volume de 1,9 litre de lait écrémé est acidifié à l'aide d'acide chlorhydrique 1N jusqu'à l'obtention d'un pH de 4,25 à la température de 22°C. La durée de cette opération est d'environ 30 minutes.

Le caséinate de sodium est précipité en culot par centrifugation à 8000 g pendant trente minutes à 22°C. Le culot de caséine, décanté du surnageant , est resolubilisé dans douze fois son poids d'une solution tampon de NaCl 0,05 M afin d'éliminer les traces de lactosérum puis fait l'objet d'une seconde étape de précipitation par acidification à l'aide d'HCl 1 N puis une nouvelle centrifugation à 8000 g pendant trente minutes à 22°C.

Le culot final est redissous dans 1,9 litre d'eau distillée ajusté à pH 7,6 à l'aide d'une solution de NaOH 1 N maintenue à ce pH pendant 60 minutes à 35°C.

La solution finale de caséinate de sodium, d'un volume de 1,49 litre, est ensuite diafiltrée contre 4 fois le volume de la solution initiale.

La stérilisation de la solution de caséinate de sodium est réalisée à l'aide de deux filtrations consécutives, respectivement:
- une première filtration à l'aide d'un dispositif de filtration équipé d'une membrane filtrante d'un diamètre de pore moyen de 0,45 µm du type « oméga » commercialisée par la Société FILTRON, cette filtration permettant d'éliminer la matière grasse résiduelle; et
- une seconde filtration à l'aide d'un dispositif équipé d'une membrane d'un diamètre de pore moyen de 0,22 µm de type « oméga » commercialisée par la Société FILTRON.

### EXEMPLE 4 :

### Analyse biochimique des différentes fractions d'un lait d'équidé.

La composition et la qualité bactériologique des fractions de lait d'équidé préparées conformément aux exemples 1 à 3 ci-dessus ont été analysées. Les résultats sont présentés dans le tableau 1.

On peut noter que la fraction de caséinate de sodium contient 4,65 g de protéine lactosérique pour 100 g de poudre, ce que représente environ 6% les protéines contenues dans le produit final. Ces protéines contaminantes ont été identifiées par chromatographie haute performance en phase inverse. Il s'agit du lyzozyme (1,5% en poids), de l'alpha-lactalbumine (2,7% en poids) et de la bêta-lactoglobuline (1,8% en poids).

### EXEMPLE 5 :

### Activité d'inhibition de la production d'interleukine-1 des différentes fractions d'un lait d'équidé selon l'invention.

### A. MATERIELS ET METHODES

Des kératinocytes humains ont été isolés à partir d'un prélèvement de peau recueilli sont après une opération de plastie abdominale réalisée chez une femme âgée de 27 ans, soit à partir de peaux de prépuces.

Les kératinocytes présents dans le prélèvement de peau ont été séparés dans une solution de Hanks (GIBCO) une nuit à 4°C. La solution est dépourvue de Ca⁺⁺ et de Mg⁺⁺ et additionnée de 1% de Penicilline et de Streptomycine, et de 0.5% de Trypsine.

Les kératinocytes ont été cultivés en culture primaire dans un milieu de type MCK dans un incubateur en atmosphère humide à 37°C contenant 5% de CO₂.

Les kératinocytes utilisés pour les tests de production d'interleukine-1 sont issus d'un troisième « repiquage » ou « passage » en milieu MCK dans les conditions précitées.

Au moment du test, le milieu MCK est remplacé par un milieu SFM complet, commercialisé par la Société GIBCO sous la référence KSFM et les cellules ont été pré-incubées dans ce milieu pendant 60 minutes à 37°C en atmosphère humide contenant 5% de CO₂ avec les produits suivants:
- des concentrations croissantes d'une poudre de lactosérum préparée conformément à l'exemple 2;
- des concentrations croissantes d'une poudre de caséinate de sodium préparée conformément à l'exemple 3;
- des concentrations croissantes d'une poudre de lait écrémé préparée conformément à l'exemple 1.

Les cellules préincubéés pendant 60 minutes avec chacun des produits ci-dessus ont été irradiées par un rayonnement ultraviolet de type UVB à la dose de 0,1 J/cm². L'incubation des cellules préincubées avec les produits à tester et irradiée comme indiqué ci-avant a été poursuivie pendant dix huit heures à 37°C en atmosphère humide contenant 5% de CO₂.

En outre, des cultures cellulaires témoins irradiées et non-irradiées ont été réalisées en l'absence de préincubation avec l'un quelconque des produits à tester.

A la fin de l'incubation, la quantité d'interleukine-1 α présente dans les milieux de culture a été mesurée à l'aide d'un kit de diagnostic de type ELISA commercialisé par la Société R & D SYSTEMS, sous la référence « Human II-1α immuno assay (catalog number DLA 50) ».

Parallèlement, la teneur en protéines a été dosée par la méthode au bleu de coomassie décrite par BRADFORD (1996). A mol Biochemistry 1976 - 248-254.

### B. RESULTATS

Les résultats de production d'interleukine-1 α sont exprimés en pg d'II-1α par mg de protéines cellulaires et sont représentés dans le tableau Il ci-après.

Au vu des résultats de production d'interleukine-1α ci-dessus, il est observé que la poudre de lactosérum inhibe la production d'interleukine-1α par les kératinocytes humains, l'activité inhibitrice étant fonction de la dose de poudre de lactosérum ajoutée. Ainsi, une inhibition de la production d'interleukine-1α de 71% par rapport aux cellules témoins est observée pour une dose de lactosérum de 0,01% en poids, par rapport au volume de la culture cellulaire et de 94% d'inhibition pour une dose de lactosérum de 0,1% en poids par rapport au volume de la culture cellulaire.

De même, une inhibition de 93% de la production d'interleukine-1α est observée avec la poudre de caséinate de sodium lorsque celle-ci est utilisée à une dose de 0.0001% en poids par rapport au volume de la culture cellulaire.

A des doses supérieures, la poudre de caséinate de sodium se révèle cytotoxique; il a en effet été observé une diminution respectivement de 77% et de 85% de la teneur en protéines kératinocytaires pour des doses de 0,1 et de 1% (p/v) de caséinate de sodium.

Une inhibition de la production d'interleukine-1α par les kératénocytes de l'ordre de 54% a été observée avec une dose de 0,01% (p/v) de poudre de lait écrémé. Aux doses supérieures, la poudre de lait écrémé est cytotoxique. En effet, pour une dose de 1% (p/v) de poudre de lait écrémé, on observe une diminution de 89% de la teneur en protéines kératinocytaires.

Les résultats obtenus ci-dessus montrent clairement que les fractions de lait d'équidé, du fait de leur capacité à inhiber la production d'interleukine-1α par les cellules, exercent un effet anti-inflammatoire.

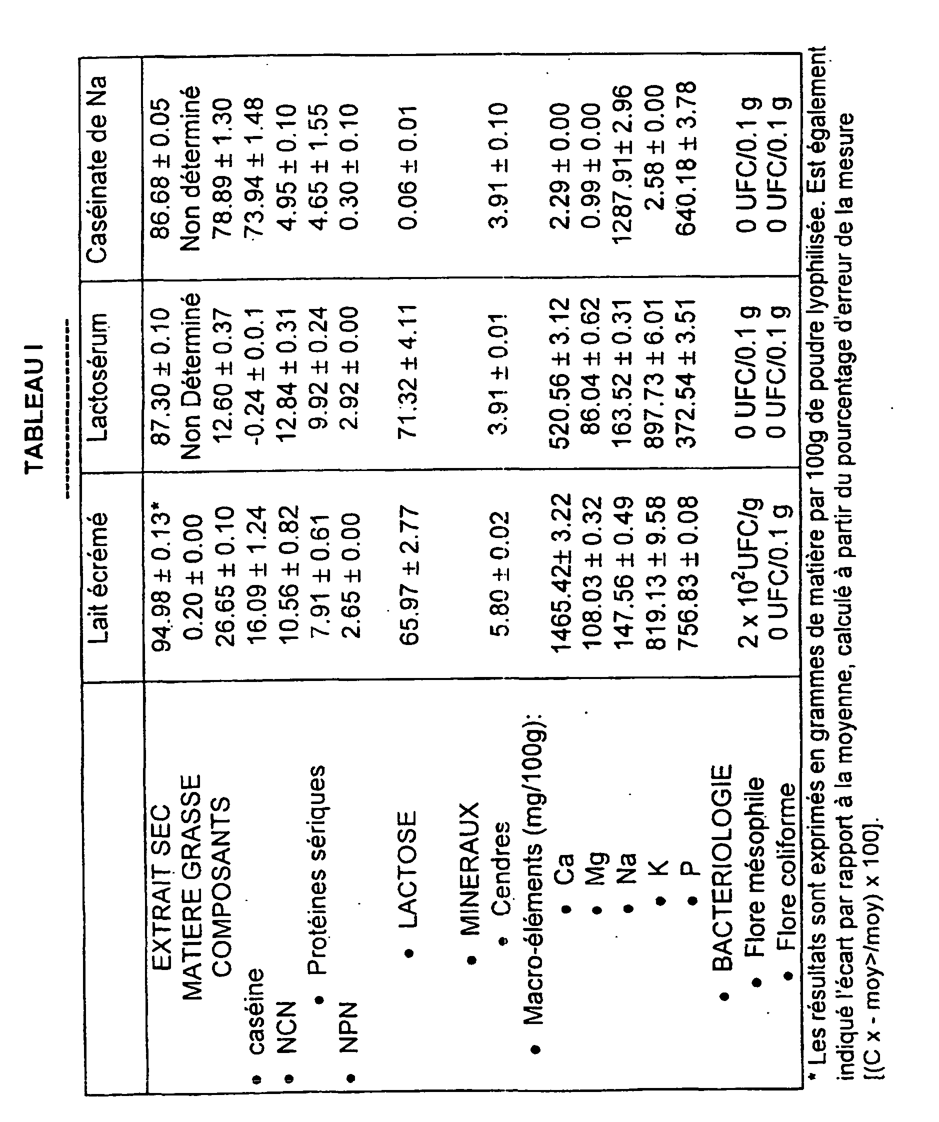

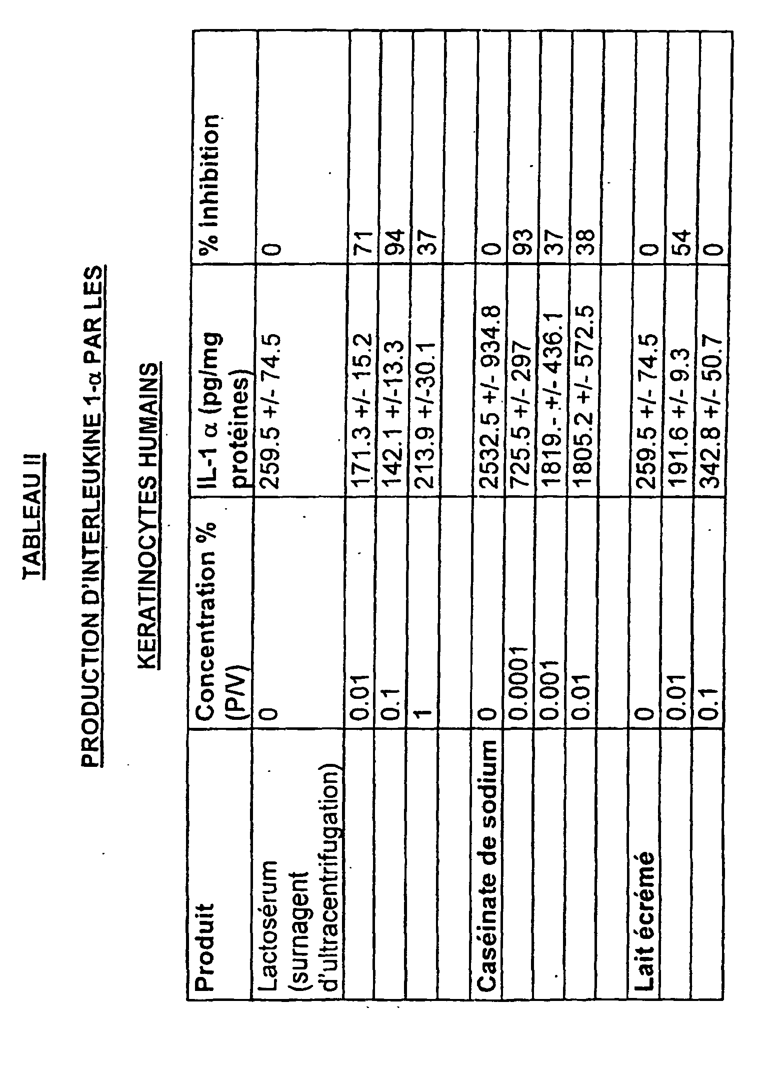

## Revendications

1. Utilisation d'une fraction du lait d'équidé choisie parmi du lait écrémé, du lactosérum et du caséinate de sodium pour la fabrication d'une composition possédant une activité anti-inflammatoire.

2. Utilisation selon la revendication 1, **caractérisée en ce qu'**il s'agit de lait écrémé.

3. Utilisation selon la revendication 1, **caractérisée en ce qu'**il s'agit de lactosérum, préférentiellement d'un lactosérum doux.

4. Utilisation selon la revendication 1, **caractérisée en ce qu'**il s'agit de caséinate de sodium.

5. Utilisation selon l'une des revendications 1 à 4, **caractérisée en ce que** la composition est une composition pharmaceutique, une composition cosmétique ou une composition alimentaire fonctionnelle.

6. Composition pharmaceutique, cosmétique ou destinée à l'alimentation de l'homme ou de l'animal **caractérisée en ce qu'**elle comprend une fraction du lait d'équité choisie parmi du lait écrémé, du lactosérum et du caséinate de sodium et possède une activité anti-inflammatoire.

7. Composition selon la revendication 6, **caractérisée en ce qu'**il s'agit d'une composition pharmaceutique.

8. Composition selon la revendication 6, **caractérisée en ce qu'**il s'agit d'une composition cosmétique.

9. Composition selon la revendication 6, **caractérisée en ce qu'**il s'agit d'une composition pour l'alimentation fonctionnelle de l'homme ou de l'animal.

## Claims

1. A use of a fraction of equine milk selected among skimmed milk, lactoserum and sodium caseinate for the preparation of a composition having anti-inflammatory activity.

2. A use according to claim 1, **characterised in that** it is skimmed milk.

3. A use according to claim 1, **characterised in that** it is lactoserum, preferably soft lactoserum.

4. A use according to claim 1, **characterised in that** it is sodium caseinate.

5. A use according to any one of claims 1 to 4, **characterised in that** the composition is a pharmaceutical composition, a cosmetic composition or a functional food composition.

6. A pharmaceutical, cosmetic composition or intended as food for man or animal, **characterised in that** it comprises a fraction of equine milk selected among skimmed milk, lactoserum and sodium caseinate and having an anti-inflammatory activity.

7. A composition according to claim 6, **characterised in that** it is a pharmaceutical composition.

8. A composition according to claim 6, **characterised in that** it is a cosmetic composition.

9. A composition according to claim 6, **characterised in that** it is a composition for functional food of man or animal.

## Patentansprüche

1. Verwendung einer Fraktion aus Milch von Pferdeartigen ausgewählt aus Magermilch, Molke und Natriumcaseinat zur Herstellung einer Zusammensetzung, die eine entzündungshemmende Wirkung aufweist.

2. Verwendung nach Anspruch 1, **dadurch gekennzeichnet, dass** es sich um Magermilch handelt.

3. Verwendung nach Anspruch 1, **dadurch gekennzeichnet, dass** es sich um Molke handelt, vorzugsweise um Süßmolke.

4. Verwendung nach Anspruch 1, **dadurch gekennzeichnet, dass** es sich um Natriumcaseinat handelt.

5. Verwendung nach einem der Ansprüche 1 bis 4, **dadurch gekennzeichnet, dass** die Zusammensetzung eine pharmazeutische Zusammensetzung, eine kosmetische Zusammensetzung oder eine funktionelle Nahrungszusammensetzung ist.

6. Pharmazeutische, kosmetische oder zur Ernährung von Mensch oder Tier bestimmte Zusammensetzung **dadurch gekennzeichnet, dass** sie eine Fraktion aus Milch von Pferdeartigen umfasst, die ausgewählt ist aus Magermilch, Molke und Natriumcaseinat und eine entzündungshemmende Wirkung aufweist.

7. Zusammensetzung nach Anspruch 6, **dadurch gekennzeichnet, dass** es sich um eine pharmazeutische Zusammensetzung handelt.

8. Zusammensetzung nach Anspruch 6, **dadurch gekennzeichnet, dass** es sich um eine kosmetische Zusammensetzung handelt.

9. Zusammensetzung nach Anspruch 6, **dadurch gekennzeichnet, dass** es sich um eine Zusammensetzung zur funktionellen Ernährung von Mensch oder Tier handelt.
